# DEMANDE DE BREVET EUROPEEN

(11) **EP 0 712 625 A1**
(43) Date de publication de la demande: **22.05.1996**
(21) Numéro de dépôt: 95402459.2
(22) Date de dépôt: 03.11.1995
(51) Int. Cl.: A61K 7/42

(54) **Nouvelles compositions cosmétiques autobronzantes**

(30) Priorité: 15.11.1994 FR 9413664
(71) Demandeur: Shiseido International France SA, F-75008 Paris (FR)
(72) Inventeur: Leclere, Jacques, F-77186 Noisiel (FR)
(74) Mandataire: Rinuy, Santarelli

(57) **Abrégé**

Composition cosmétique autobronzante, renfermant pondéralement de 0,05 % à 0,5 % de lawsone, de 0,05 à 5 % d'extrait hydro ou liposoluble de noix, et 10 à 30 % de DHA microencapsulée.

## Description

La présente invention concerne de nouvelles compositions cosmétiques autobronzantes.

On connaît déjà des compositions cosmétiques produisant un bronzage artificiel de la peau, en l'absence de soleil ; ces produits seront appelés ci-après "autobronzants". Ils renferment principalement de la dihydroxy acétone, ci-après dénommée DHA.

Avec la DHA, une coloration nécessite une heure avant d'apparaître, avec un maximum atteint après deux heures environ. En outre, la DHA procure à la peau une couleur jaune - orangée, qui n'est pas très naturelle.

On connaît également des produits autobronzants teintés, qui utilisent outre la DHA qui prend le relais après quelques heures, soit des colorants solubles, soit des pigments minéraux procurant un maquillage de la peau. Ceux-ci laissent des traces s'ils sont frottés par un vêtement par exemple.

Il serait donc souhaitable de disposer de produits cosmétiques capables de procurer à la peau une coloration rapide, avec une bonne tenue dans le temps et procurant également la coloration la plus naturelle possible.

C'est pourquoi la présente invention a pour objet une composition cosmétique autobronzante, caractérisée en ce qu'elle renferme pondéralement de 0,05 % à 0,5 % de lawsone, de 0,05 à 5 % d'extrait hydro- ou liposoluble de noix, et 10 à 30 % de DHA microencapsulée. Cette dernière proportion concerne une DHA microencapsulée renfermant 20 % de DHA pure.

Cette composition cosmétique peut prendre toutes les formes classiques des produits cosmétiques à usage dermatologique, telles que mousse, crème, lait, gel, etc ...

Dans les compositions cosmétiques ci-dessus, la proportion de la lawsone peut aller de 0,05 à 0,5 % en poids, et se trouve de préférence dans une proportion d'environ 0,1 % en poids. La lawsone est commercialisée par exemple par les Sociétés Alban-Müller ou Sochibo.

L'extrait de noix hydro-, et de préférence liposoluble peut être présent dans une proportion de 0,05 à 5 % dans les compositions autobronzantes selon l'invention et de préférence en proportion de 3 % environ. Les extraits liposolubles (huileux) de noix ou aqueux de noix sont commercialisés par exemple par la Société Croda.

La DHA microencapsulée est commercialisée par exemple par les Laboratoires Sérobiologiques, la DHA étant alors enrobée à l'aide d'un polyméthacrylate, de telle sorte que la teneur en DHA des microcapsules soit de 20 %. Elle peut être encapsulée avec tout polymère de micro encapsulation classique compatible permettant une bonne étanchéité de la microcapsule.

On trouve de préférence de 10 à 30 % de DHA microencapsulée (soit 2 % à 6 % de DHA pure), et notamment environ 20 %, dans les compositions cosmétiques autobronzantes selon l'invention.

Les compositions autobronzantes selon la présente invention permettent d'obtenir une coloration rapide et durable de la peau ; de plus la teinte obtenue est agréable et naturelle.

En outre, il est à noter que les compositions selon la présente invention remplissent également la fonction de filtre solaire.

Elles trouvent donc tout naturellement leur application en cosmétique notamment dans le maquillage et les protections des radiations ultraviolettes.

Des compositions autobronzantes préférées selon l'invention renferment en outre 0,05 % à 0,5 % en poids d'hématéine et de préférence environ 0,15 %. Celle-ci est généralement extraite du bois de Panama (Heamatoxylon campechianum L) et est commercialisée par exemple par la Société Laserson.

L'hématéine ne modifie pas la rapidité de coloration de la peau procurée par les autres constituants du mélange, tout en permettant une coloration immédiate de celle-ci.

Le produit cosmétique fini doit avoir un pH compris entre 2,5 et 5,5 et de préférence environ 4,5. L'ajustement du pH peut être obtenu avec les acides et bases classiques acceptables en cosmétologie.

Les compositions cosmétiques selon l'invention sont par exemple des mousses, les gels, des lotions, et de préférence des laits ou des crèmes.

Outre les composés cités ci-dessus, elles renferment les excipients classiques des préparations dermatologiques tels que ceux cités dans les exemples.

Elles peuvent être préparées classiquement par mélange des différents constituants selon les méthodes appropriées.

La présente demande a donc aussi pour objet un procédé de préparation d'une composition cosmétique ci-dessus, caractérisant en ce que l'on mélange, en proportions pondérales, de 0,05 % à 0,5 % de lawsone, de 0,05 à 5 % d'extrait hydro- ou liposoluble de noix, et 10 à 30 % de DHA microencapsulée.

Dans des conditions préférentielles de mise en oeuvre du procédé, on mélange les composants ci-dessus dans les proportions préférées précédemment précisées, particulièrement celles indiquées dans les exemples, et selon les procédures indiquées dans ceux-ci.

L'invention a enfin pour objet un lait autobronzant tel que défini ci-dessus, caractérisé en ce qu'il renferme 3 % d'extrait liposoluble de noix, 0,1 % de lawsone, 20 % de dihydroxyacétone microencapsulée à 20 % de dihydroxyacétone pure, et optionnellement 0,15 % d'hématéine ainsi qu'une crème autobronzante telle que définie ci-dessus, caractérisée en ce qu'elle renferme 3 % d'extrait liposoluble de noix, 0,1 % de lawsone, 20 % de dihydroxyacétone microencapsulée à 20 % de dihydroxyacétone pure, et optionnellement 0,20 % d'hématéine.

Les exemples qui suivent illustrent la présente invention. Les quantités sont exprimées en pourcentages pondéraux.

### EXEMPLE 1 : Lait autobronzant

On a préparé un lait autobronzant à partir de plusieurs phases A, B, C, D, E et F, la phase A étant composée de deux phases A1 et A2.

| | | |
|---|---|---|
| A1 : | Glycérine | 5,000 |
| | Butylène glycol | 2,000 |
| | Hématéine (Laserson) | 0,150 |
| | Mélange de parahydrobenzoates | 0,300 |
| | Phénoxétol | 0,520 |
| | EDTA trisodique | 0,104 |
| | Gomme Xanthane | 0,500 |
| A2 : | Carbopol en solution à 1 % (Carbopol 940) | 8,000 |
| B : | PEG 30 glycéryl stéarate | 5,000 |
| | Stéarate de glycéryle | 3,500 |
| | Huile de vaseline | 3,000 |
| | Extrait liposoluble de noix (Croda) | 3,000 |
| | Solution à 1 % de Lawsone | 10,000 |
| | Malate de diisostéaryle | 2,000 |
| | Distéarate de glycéryle | 2,000 |
| | Diméthicone | 2,000 |
| | Alcool cétylique | 2,000 |
| C : | Hydroxyde de sodium | 0,015 |
| | Eau déminéralisée | 7,000 |
| D : | DHA microencapsulée dans un polyméthacrylate à 20 % de DHA (laboratoires Sérobiologiques) | 20,000 |
| E : | Acide citrique en solution à 10 % | QSP pH 4,5 à 5,5 |
| F : | Parfum | 0,200 |
| | Eau déminéralisée | QSP 100 |

### MODE OPERATOIRE

On porte les phases A et B à 70°C séparément, puis on introduit la phase B dans la phase A sous agitation pendant 30 mn à température constante, on refroidit à 50°C, ajoute la phase C, on refroidit à 45°C et introduit la phase D, on ajuste le pH avec E et, si désiré, on ajoute le parfum à 35°C.

La solution de lawsone est préparée comme suit : la lawsone est dissoute sous agitation dans le Miglyol 812 (glycéride caprique / caprilique) à environ 60°C puis refroidie.

### EXEMPLE 2 : Crème autobronzante

On a préparé une crème autobronzante à partir de plusieurs phases A, B, C, D et E.

| | | |
|---|---|---|
| A : | Glycérine | 5,000 |
| | Butylène glycol | 3,000 |
| | Hématéine | 0,200 |
| | Mélange de parahydroxybenzoates (Laserson) | 0,300 |
| | Phénoxétol | 0,500 |
| | EDTA trisodique | 0,100 |
| B : | Alcool cétylique / stéarylique A.E | 10,000 |
| | Huile de vaseline | 3,000 |
| | Extrait liposoluble de noix | 3,000 |
| | Solution à 1 % de Lawsone | 10,000 |
| | Huile de sésame vierge | 5,000 |
| C : | DHA microencapsulée | 20,000 |
| E : | Acide citrique en solution aqueuse à 10 % | QSP pH 4,5 à 5,5 |
| F : | Parfum | 0,200 |
| | Eau déminéralisée | QSP 100 |

### MODE OPERATOIRE

On chauffe la phase A à 70°C jusqu'à dissolution, chauffe également la phase B à 70°C, mélange sous agitation à 70°C pendant 20 mn, refroidit à 45°C, introduit la phase C, ajuste le pH avec la phase E, refroidit à 35°C et ajoute alors le parfum si désiré.

### EXEMPLE 3 : Gel crème autobronzant

On a préparé un gel crème autobronzant à partir de plusieurs phases A, B, C, D, E et F.

| | | |
|---|---|---|
| A : | Glycérine | 4,500 |
| | Butylène glycol | 3,000 |
| | Propylène glycol | 1,500 |
| | Mélange de parahydroxybenzoates | 0,250 |
| | Alcool éthylique | 7,500 |
| | Carbopol 940 à 2 % | 50,000 |
| B : | PEG 23 lauryl éther | 3,500 |
| | Extrait liposoluble de noix | 3,000 |
| | Solution à 1 % de Lawsone | 10,000 |
| C : | Hydroxyde de sodium | 0,020 |
| | Eau déminéralisée | 1,000 |
| D : | DHA microencapsulée | 15,000 |
| E : | Acide citrique solution à 10 % | QSP pH 5,5 |
| F : | Parfum | 0,200 |
| | Eau déminéralisée | QSP 100 |

Ce gel est préparé comme suit : on fait un gel avec la phase A, dissout le PEG 23 Lauryl éther sous agitation à 50°C, ajoute les composants huileux constituant la phase B à la même température, neutralise à l'hydroxyde de sodium, refroidit à 40°C, ajoute la DHA microencapsulée et règle le pH à l'aide de la phase D. Si désiré on ajoute le parfum.

### EXEMPLE 4 : Gel crème autobronzant

On a préparé un gel analogue au gel ci-dessus, comportant en outre 0,15 % d'hématéine (incorporée dans la phase hydro/alcoolique) et en remplaçant l'extrait liposoluble de noix par un extrait de noix hydroglycolique.

Par l'utilisation d'un des produits cosmétiques autobronzants ci-dessus, on obtient une coloration immédiate de la peau, se développant pendant deux heures, puis durable.

Contrairement aux produits actuellement sur le marché, une teinte de peau plus intense que celle de l'application primaire apparaît au bout de 30 minutes, au lieu d'une heure pour les produits habituels, ce qui permet un maquillage tardif.

## Revendications

1. Composition cosmétique autobronzante, caractérisée en ce qu'elle renferme pondéralement de 0,05 % à 0,5 % de lawsone, de 0,05 à 5 % d'extrait hydro ou liposoluble de noix, et 10 à 30 % de dihydroxyacétone microencapsulée et en ce que son pH est compris entre 2,5 et 5,5.

2. Composition selon la revendication 1, caractérisée en ce que la proportion de la lawsone est d'environ 0,1 % en poids.

3. Composition selon la revendication 1 ou 2, caractérisée en ce que L'extrait de noix liposoluble est en proportion de 3 % environ.

4. Composition selon l'une des revendications 1 à 3, caractérisée en ce que la dihydroxyacétone est enrobée à l'aide d'un polyméthacrylate, de telle sorte que la teneur en dihydroxyacétone des microcapsules est de 20 %.

5. Composition selon l'une des revendications 1 à 4, caractérisée en ce que la proportion de dihydroxyacétone microencapsulée exprimée en dihydroxyacétone pure est d'environ 4 %.

6. Composition selon l'une des revendications 1 à 5, caractérisée en ce qu'elle renferme en outre 0,05 % à 0,5 % d'hématéine.

7. Composition selon l'une des revendications 1 à 6, caractérisée en ce qu'elle se présente sous forme de lait, crème ou gel.

8. Lait autobronzant selon la revendication 7, caractérisé en ce qu'il renferme 3 % d'extrait liposoluble de noix, 0,1 % de lawsone, 20 % de dihydroxyacétone microencapsulée à 20 % de dihydroxyacétone pure, et optionnellement 0,15 % d'hématéine.

9. Crème autobronzante selon la revendication 7, caractérisée en ce qu'elle renferme 3 % d'extrait liposoluble de noix, 0,1 % de lawsone, 20 % de dihydroxyacétone microencapsulée à 20 % de dihydroxyacétone pure, et optionnellement 0,20 % d'hématéine.

10. Procédé de préparation d'une composition selon l'une de revendications 1 à 9, caractérisé en ce que l'on mélange, selon des méthodes connues en elles-mêmes, les constituants dans les proportions appropriées.
